# EUROPEAN PATENT APPLICATION

(11) **EP 2 711 712 A1**
(43) Date of publication of application: **26.03.2014**
(21) Application number: 13177734.4
(22) Date of filing: 24.07.2013
(51) Int. Cl.: G01N 33/92

(54) **Composition and kit for isolating vesicles and method of isolating the vesicles using the same**

(30) Priority: 19.09.2012 KR 20120104216
(71) Applicant: Samsung Electronics Co., Ltd, Gyeonggi-do 443-742 (KR)
(72) Inventor: Yong, Ye-ryoung, Gyeonggi-do (KR); Kang, Hyun-ju, Gyeonggi-do (KR)
(74) Representative: Grünecker, Kinkeldey, Stockmair & Schwanhäusser

(57) **Abstract**

The invention provides compositions, kits, and methods for analyzing vesicles or vesicle proteins, glycoproteins, lipids or nucleic acids, and may be used for screening ligands that have a binding affinity to vesicles.

## Description

### BACKGROUND

### 1. Field

The present disclosure relates to compositions and kits for isolating vesicles, and methods of isolating vesicles using the same.

### 2. Description of the Related Art

*In vivo* microvesicles are small membranous vesicles that exist in or are secreted from various cell types. Microvesicles secreted from cells include: (i) exosomes, which are membranous vesicles that originate from phagocytic cells and have a diameter of 30 to 100 nm; (ii) ectosomes (also called shedding microvesicles (SMVs)), which are membranous vesicles that are released from the plasma membrane and have a diameter of 50 to 1000 nm; and (iii) apoptotic blebs, which are vesicles that are secreted from dying cells and have a diameter of 50 to 5000 nm.

Electron microscopy has confirmed that exosomes are not separated directly from a plasma membrane, but originate in intracellular regions called multivesicular bodies (MVBs). Multivesicular bodies fuse with the plasma membrane and are then released from the cells as exosomes. Exosomes are released from a plurality of cell types under normal and/or pathologic states. Although the molecular mechanisms of exosomes are unknown, it is known that, in addition to red blood cells, various kinds of immune cells, such as B-lymphocyte, T-lymphocyte, dendritic cells, blood platelets, and macrophage, and tumor cells, produce and secrete exosomes. *In vivo* microvesicles, such as exosomes, may contain microRNA (miRNA), which may be used as a marker in molecular diagnosis, including the diagnosis of cancer, hereditary disease, heart disease, or neuronal disease, such as schizophrenia.

Previous methods of isolating microvesicles involve immuno-capture of microvesicles using antibodies. However, such methods are limited due to protein conformational changes, microvesicle heterogeneity, protein interactions, target detection, etc. Also, separation or detection of microvesicles may require a complicated process or a high-cost apparatus.

Therefore, there is a need for improved methods of isolating microvesicles, analyzing microvesicles, and screening ligands that bind to microvesicles, as well as compositions and a kit for isolating vesicles that overcome the above drawbacks.

### SUMMARY

Provided is a composition for isolating a vesicle, the composition comprising a first component linked to a lipophilic molecule that can intervene into a lipid bilayer; and a second component that binds to the first component.

Also provided is a kit for isolating a vesicle, the kit comprising a first component linked to a lipophilic molecule that can intervene into a lipid bilayer; and a second component that binds to the first component.

Further provided is a method of isolating a vesicle in a sample, the method comprising incubating a first component linked to a lipophilic molecule that can intervene into a lipid bilayer with a sample comprising a vesicle, such that the lipophilic molecule embeds in lipid bilayer of the vesicle; incubating the resultant reaction mixture with a second component that binds to the first component; and separating the vesicle from the resultant reaction mixture.

Additional aspects are defined in the dependent claims and will be set forth in part in the description which follows and, in part, will be apparent from the description, or may be learned by practice of the presented embodiments.

### BRIEF DESCRIPTION OF THE DRAWINGS

These and/or other aspects will become apparent and more readily appreciated from the following description of the embodiments, taken in conjunction with the accompanying drawings in which:

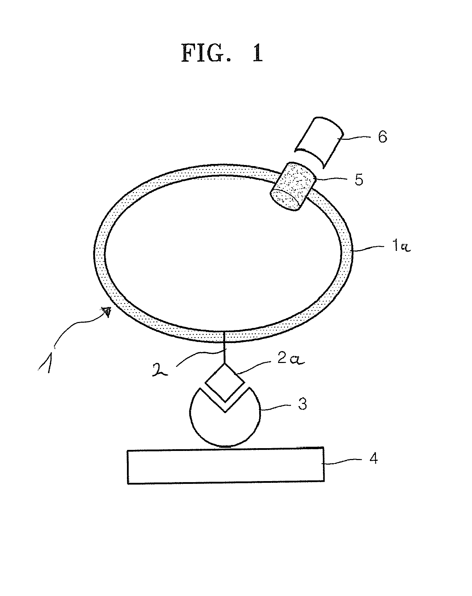

FIG. 1 is a schematic drawing that illustrates a method of isolating vesicles using a a first component linked to a lipophilic molecule capable of embedding in a lipid bilayer of a vesicle, and a second component that binds to the first component (1: vesicle, 1a: lipid bilayer of the vesicle, 2: lipophilic molecule, 2a: first component linked to lipophilic molecule, 3: second component, 4: solid support, 5: surface protein of vesicle, 6: ligand capable of binding to the surface protein of the vesicle);

FIGS. 2A and 2B are graphs that illustrate isolating microvesicles from blood plasma; and

FIG. 3 is a graph that illustrates the analysis of a surface protein of isolated microvesicles.

FIG. 4 is a graph that illustrates the screening of a ligand for binding affinity to a microvesicle.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Provided is a composition for isolating a vesicle, the composition including a first component linked to a lipophilic molecule capable of embedding in a lipid bilayer of the vesicle and a second component capable of binding to the first component.

The "lipophilic molecule" is a material that is soluble in fat, oil, lipid, and nonpolar solvents. For example, the lipophilic molecule may be a fatty acid, sterol, glyceride, or phospholipid. A phospholipid, a major component of the cell membrane, is a lipid that has a phosphate ester. Non-limiting examples of phospholipids include phosphatidate, cephalin, lecithin, phosphatidyl serine, phosphoinositide, sphingomyelin, 1,2-dihexadecanoyl-sn-glycero-3-phosphoethanolamine (DHPE), 1,2-dipalmitoyl-sn-glycero-3-phosphoethanolamine-N-(2,4-dinitrophenyl), or 1,2-dipalmitoyl-sn-glycero-3-phosphoethanolamine-N-[6-[(2,4-dinitrophenyl)amino]hexanoyl].

"First component" refers to a material that has a binding affinity to the second component. The first component may be, for example, an antibody, an antigen, a hapten, an enzyme, an enzyme substrate, an enzyme inhibitor, biotin, or avidin. The first component is linked to the lipophilic molecule. If the first component is a hydrophilic material, the lipophilic molecule linked with the first component may be an amphipathic molecule that has both hydrophilic and lipophilic properties in a single molecule: the hydrophilic part/property of the amphiphilic molecule for linking with the first component and the lipophilic part/property for constituting the lipophilic molecule of the invention. Thus, the lipophilic molecule defined in the claims may be an amphipathic molecule if it has its lipophilic part exposed to outside, such that it can introduce a bilayer, if linked with the first compound.

The lipophilic molecule may intervene (e.g. embed) in a lipid bilayer. The lipid bilayer may be, for example, a lipid bilayer of a liposome, a cell membrane, a vesicle, a microvesicle, or an exosome.

"Second component" refers to a material that has a binding affinity to the first component. For example, if the first component is an antibody, the second component may be an antigen that binds the antibody. If the first component is an antigen, the second component may be an antibody for the antigen, and vice versa. Similarly, if the first component is a hapten, the second component may be a hapten-binding protein, or vice versa. If the first component is an enzyme, the second component may be a substrate or an inhibitor of the enzyme, or vice versa. If the first component is biotin, the second component may be avidin; or vice versa, if the first component is avidin, the second component may be biotin. The first component may be arranged on a water soluble surface of the vesicle when the lipophilic molecule intervenes into the bilayer, such that the second component binds the first component to form a complex comprising the vesicle, the first component, and the second component.

"Vesicle" refers to a membranous structure surrounded by a lipid bilayer. For example, the vesicle may be a liposome or a microvesicle. "Microvesicle" refers to a small membranous vesicle having a lipid bilayer and originating from a cell. Herein, the term "microvesicle" may be interchangeably used with the terms "circulating microvesicle" or "microparticle." Microvesicles may exist in cells or may be secreted from cells. Microvesicles secreted from cells may include exosomes, ectosomes (shedding microvesicles (SMVs)), apoptotic blebs, or any combinations thereof. The exosomes may be membranous vesicles about 30 to about 100 nm in diameter that originate from cells. The ectosomes (SMVs) may be large membranous vesicles of about 50 to about 1000 nm diameter that are directly leaked from plasma membranes. The apoptotic blebs may be vesicles about 50 to about 5000 nm in diameter that are secreted from dying cells. *In vivo* microvesicles may contain microRNAs (miRNAs) or messenger RNAs (mRNAs). Surface proteins of the microvesicles may be disease-specific markers.

Also provided is a kit for isolating a vesicle including a first component linked to a lipophilic molecule capable of embedding in a lipid bilayer of the vesicle and a second component capable of binding to the first component. The kit may be provided in any suitable form. For instance, the first component linked to the lipophilic molecule and the second component may be packaged in separate containers, or in a single, divided container. Furthermore, the kit may include additional components typically used in the isolation of vesicles, for instance one or more buffers, reagents for separating and/or detecting the vesicle such as a solid support, to which the second component may be bound or which is capable of binding the second component, as well as one or more ligands that are capable of binding to a vesicle, or reagents for amplifying and/or analyzing a nucleic acid. At least one of the afore-said may also be included in the composition of the composition of the invention. All other aspects of the kit, including the lipophilic molecule, first component, second component, and vesicle, are as previously described.

Also provided is a method of isolating a vesicle including (1) incubating a first component linked to a lipophilic molecule capable of intervening or embedding in a lipid bilayer of the vesicle with a sample comprising a vesicle, such that the lipophilic molecule intervenes into or becomes embedded in the lipid bilayer of the vesicle and, thus, attached thereto, (2) incubating the resultant reaction mixture with a second component that binds to the first component which is bound to the vesiclet, and (3) separating the vesicle from the resultant reaction mixture. The first component linked to a lipophilic molecule, second component, and vesicle are as previously described.

The sample may be a body fluid sample or cell culture sample. The body fluid may be, for example, urine, mucus, saliva, tears, blood plasma, blood serum, sputum, spinal fluid, serous fluid from a pleural cavity (e.g., a sample from a hydrothorax condition), nipple aspirate, lymph, tracheolar fluid, intestinal juice, genitourinary tract fluid, breast milk, semen, peritoneal fluid (e.g., a sample from ascites), cystic tumor fluid, amniotic fluid, or any combination thereof.

The sample may be free of normal cells, dead cells, or cell debris. For example, the sample may be pretreated with centrifugation, dialysis, or any combinations thereof.

The incubation may be performed by any suitable technique. The incubating may be performed *in vitro.* For example, the incubating may be performed at room temperature and/or while mixing reactants.

In any of the foregoing embodiments of a composition, kit, or method, the second component may be fixed on a solid support. The solid support may be, for example, a plate or a bead, such as a polystyrene plate or a polystyrene bead.

The separating may be achieved by, for example, removal of the reaction mixture, washing, or any combinations thereof. The separating may include separating the vesicle from the second component fixed to the solid support, such as by incubating the reaction mixture with an additional amount of the second component that is not fixed to the solid support.

The method may further include detecting the separated vesicle. The detecting may be performed by any method known in the art. For example, the detecting may be performed by staining the vesicle, observing the vesicle with an electronic microscope, or using a ligand linked to a fluorescent material. For example, when the fluorescent material is a fluorescent protein, the intensity of the fluorescent light that is generated under illumination with ultraviolet rays may be measured using a fluorophotometer.

The method may further include contacting the separated vesicle with a ligand having a binding affinity to the vesicle (e.g., binding affinity to a feature on the surface of the vesicle other than the first component bound to the lipophilic molecule), and detecting the ligand bound to the vesicle to analyze the vesicle. The ligand may, for example, have a binding affinity for protein, glycoprotein, phospholipid or cholesterol. The ligand may be, for example, a material having a binding affinity for protein, an enzyme substrate, a coenzyme, a regulatory factor, a material that specifically binds with receptors, lectin, an antigen, an antibody, a hormone, a neurotransmitter, a phospholipid-binding protein, a protein that includes pleckstrin homology (PH) domain, or a cholesterol-binding protein. The ligand may be linked to a fluorescent material. For example, when the fluorescent material is a fluorescent protein, the intensity of fluorescence that is generated under illumination with ultraviolet rays may be measured using a fluorophotometer. The vesicle may be analyzed by detecting the ligand bound to the vesicle.

The method may further include separating a nucleic acid from the vesicle by dissolving or otherwise rupturing the separated vesicle; and, optionally, amplifying the separated nucleic acid to analyze the nucleic acid of the vesicle. The dissolving or rupturing of the vesicle may be performed by any suitable technique, such as in the presence of, for example, a chaotropic salt, an organic solvent, or a solvent that includes a surfactant. The dissolving of the vesicle may be performed with, for example, heating, mixing, rotating, vortexing, or any combinations thereof. "Nucleic acid" refers to a polymer material comprising a purine base or a pyrimidine base, sugar, and phosphate. The nucleic acid may be, for example, mRNA or miRNA. For example, the nucleic acid may be amplified by a reverse-transcription polymerase chain reaction. The amplified nucleic acids may be analyzed to analyze the nucleic acid of the vesicle.

The method may further include contacting two or more ligands to the separated vesicle, and detecting the ligands bound to the vesicle to screen the ligands. The ligand may, for example, have a binding affinity for protein, glycoprotein, or lipid. The ligand may be, for example, a material having a binding affinity for proteins, an enzyme substrate, a coenzyme, a control factor, a material that specifically binds with receptors, a lectin, an antigen, an antibody, a hormone, a neurotransmitter, a phospholipid-binding protein, a protein that includes pleckstrin homology (PH) domain, or cholesterol-binding protein. The ligand may be linked with, preferably bound to a fluorescent material. The screening of the ligands for affinity to the vesicles may be performed by comparing values of signals measured from a control group or values of signals measured from other types of ligands. The method may further include performing a washing after contacting two or more ligands to the vesicle. By comparing the measured signal values, the ligands having a binding affinity to the microvesicle may be screened.

The compositions, kits, and methods described herein may be used for any purpose, such as for analyzing vesicles, or proteins, glycoproteins, lipids or nucleic acids thereof, and may be used for screening ligands that have a binding affinity to the vesicles.

### EXAMPLES

### Example 1. Separation of microvesicles

1 µl of blood plasma (blood plasma of a stage 0 breast cancer patient) and 40 pmol of biotin-X DHPE (N-((6-(biotinoyl)amino)hexanoyl)-1,2-dihexadecanoyl-sn-glycero-3-phosphoethanolamine, triethylammonium salt) were mixed, and incubation was performed for 30 minutes, 1 hour, or 3 hours at 4°C. In this embodiment, biotin is the first component linked to the lipophilic material DHPE. The incubated blood plasma and biotin-X DHPE were added to a plate with streptavidin (the second component in this embodiment) fixed thereon, and incubation was performed for 1 hour at 25°C. Then, calcein-acetoxymethyl ester (calcein-AM) was added for detecting the complex of streptavidin-biotin-X DHPE immobilized on the plate, and a fluorophotometer (Beckman Coulter DTX 800) was used to measure the intensity of the fluorescence.

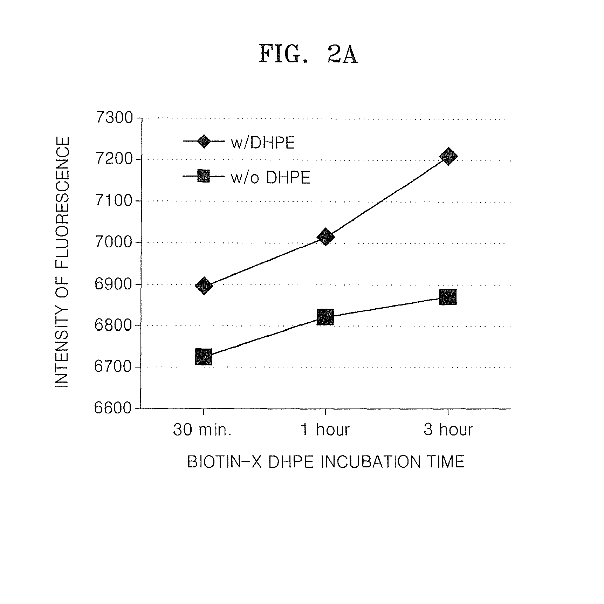

The microvesicles were separated as shown in FIG. 2A (■: no biotin-X DHPE, □: added biotin-X DHPE). FIG. 2A shows that the isolation can be improved using the present invention.

10 µl of blood plasma incubated with calcein-AM (blood plasma of a stage 0 breast cancer patient) for 4 hours at 37°C was incubated with different concentrations of biotin-X DHPE (about 10 pmol to about 320 pmol) for 4 hours at 4°C. Then, incubation was performed in a plate with streptavidin fixed thereon for 1 hour at 25°C, and a fluorophotometer was used to measure the intensity of the fluorescence.

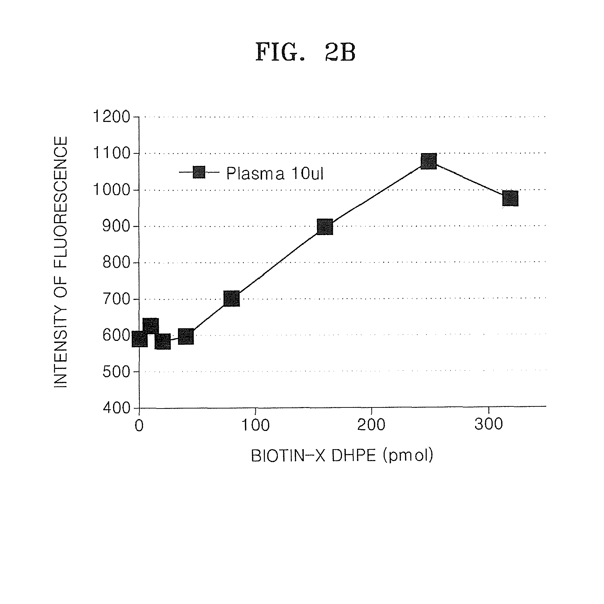

The microvesicles were separated as shown in FIG. 2B. The highest fluorescence intensity was detected when using about 150 pmol to 300 pmol, refereably about 250 pmol of biotin-X DHPE

### Example 2. Detection of nucleic acids in microvesicles

10 µl of blood plasma (blood plasma of a stage 0 breast cancer patient) and 250 pmol of biotin-X DHPE were mixed, and incubation was performed for 4 hours at 4°C. Then, this mixture was put in a plate with streptavidin fixed thereon, and incubation was performed for an hour at room temperature. The incubated solution was removed and washing was performed three times, each for 5 minutes with PBS. microRNA was extracted from the isolated microvesicles using an Invitrogen PureLink™ miRNA Isolation Kit and a reverse transcription polymerase chain reaction and a real-time polymerase chain reaction were performed with an Exiqon Universal cDNA Synthesis Kit and Exiqon microRNA Ready-to-Use PCR, Human panel I, V2.M, respectively. The result is shown in Table 1 below.

**[Table 1]**

| miRNA | Cp value of DHPE | Cp value of control group |
|---|---|---|
| hsa-miR-620 | 34.18 | >50.00 |
| hsa-miR-182 | 33.7 | >50.00 |
| hsa-miR-542-5p | 32.51 | 41.2 |
| hsa-miR-31 | 34.99 | 43.59 |
| hsa-miR-886-5p | 32.01 | 39.63 |
| hsa-miR-19a | 32.89 | 39.02 |
| hsa-miR-491-5p | 34.16 | 39.92 |
| hsa-miR-584 | 33.52 | 38.9 |
| hsa-miR-509-3p | 33.48 | 38.07 |
| hsa-miR-185* | 34.79 | 38.83 |
| hsa-miR-18b | 30.71 | 34.43 |
| hsa-miR-671-5p | 32.67 | 35.66 |
| hsa-miR-570 | 34.68 | 37.05 |
| hsa-miR-125a-5p | 32.89 | 35.22 |
| hsa-miR-526b | 33.73 | 35.96 |
| hsa-miR-30b* | 33.05 | 35.27 |

| | | |
|---|---|---|
| (* represents the opposite arm of the arm known to express relatively higher between the two arms of pre-miRNA hairpin) | | |

miRNA was detected from the separated microvesicles. 16 species among the total 375 human miRNA in an Exiqon Universal cDNA Synthesis Kit and an Exiqon microRNA Ready-to-Use PCR, Human panel I, V2.M were detected to be Cp<35. In particular, miR-18b expression was found to be elevated, corroborating reports that expression of miR-18b increases in breast cancer. Cross-point (Cp) value represents the cycle by which the fluorescence of a sample increased to a level higher than the background fluorescence in the amplification cycle, and the term "Cp value" is interchangeable with term "cycle threshold (Ct) value".

### Example 3. Detection of surface proteins in microvesicles

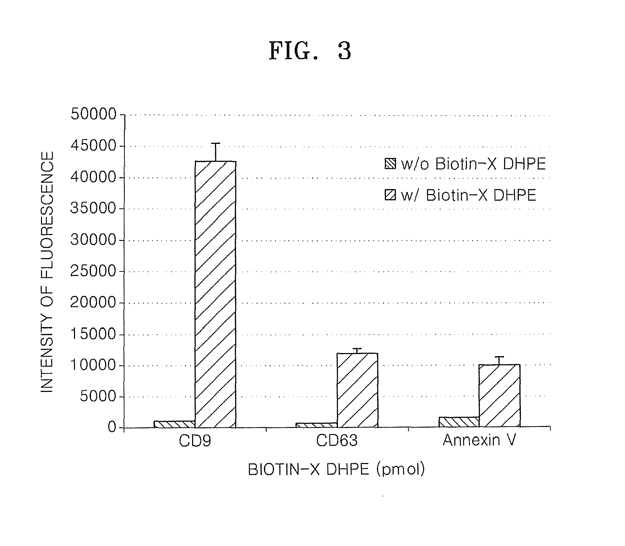

1 µl of blood plasma (blood plasma mix of normal subjects) and 1000 pmol biotin-X DHPE were mixed, and incubation was performed for 3 hours at 25°C. Then, this mixture was put in a plate with streptavidin fixed thereon, and incubation was performed for 2 hours at room temperature. The incubated solution was removed and washing was performed three times each for 5 minutes with PBS. 2 µg of anti-CD9 antibody, anti-CD63 antibody, and anti-annexin V antibody, respectively were added to the plate and a reaction was performed for 16 hours at room temperature. The antibody solutions were removed and washing was performed five times each for 3 minutes with PBS. 4 µg of Alexa Fluor® 488-conjugated secondary antibody (Invitrogen) was added, and incubation was performed for an hour at room temperature. The secondary antibody solution was removed, washing was performed 5 times each for 3 minutes with PBS, and the intensity of fluorescence was measured by using a fluorophotometer. The result is shown in FIG. 3.
It was identified that microvesicles are separated from blood plasma in the presence of biotin-X DHPE and that the used antibodies bind to the separated microvesicles. It is well known that CD9, CD63 and annexin V are surface protein markers of microvesicles.

### Example 4. Antibody screening for detection of surface proteins in microvesicles

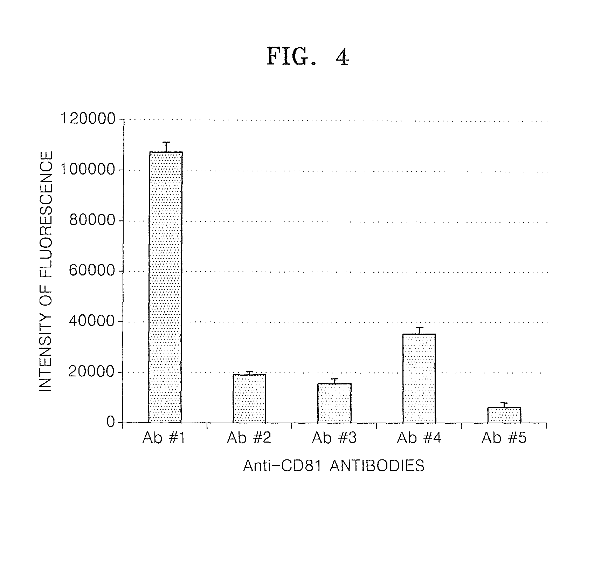

1 µl of blood plasma (blood plasma mix of normal subjects) and 1000 pmol biotin-X DHPE were mixed, and incubation was performed for 3 hours at 25°C. Then, this mixture was put in a plate with streptavidin fixed thereon, and incubation was performed for 2 hours at room temperature. The incubated solution was removed and washing was performed three times each for 5 minutes with PBS. 2 µg of anti-CD81 antibodies from different clones and suppliers were added to the plate and a reaction was performed for 16 hours at room temperature. The antibody solutions were removed and washing was performed five times each for 3 minutes with PBS. 4 µg of Alexa Fluor® 488-conjugated secondary antibody (Invitrogen) was added, and incubation was performed for an hour at room temperature. The secondary antibody solution was removed, washing was performed 5 times each for 3 minutes with PBS, and the intensity of fluorescence was measured by using a fluorophotometer. The result is shown in FIG. 4.

It was shown that antibody 1 has a high binding affinity to CD81 and a high potential for capturing microvesicles.

It was identified that the quantity of microvesicles separated from blood plasma increases and binding of antibodies also increases as the quantity of biotin-X DHPE increases. Also, CD24, CD9 and CD63, the markers of microvesicles, were detected from the separated microvesicles using biotin-X DHPE.

It should be understood that the exemplary embodiments described therein should be considered in a descriptive sense only and not for purposes of limitation. Descriptions of features or aspects within each embodiment should typically be considered as available for other similar features or aspects in other embodiments.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of isolating a vesicle in a sample, the method comprising incubating a first component linked to a lipophilic molecule that can intervene into a lipid bilayer with a sample comprising a vesicle, such that the lipophilic molecule embeds in lipid bilayer of the vesicle,
incubating the resultant reaction mixture with a second component that binds to the first component, and
separating the vesicle from the resultant reaction mixture.

2. The method of claim 1, wherein the sample is a body fluid or a cell culture.

3. The method of claim 2, wherein the body fluid is urine, mucus, saliva, tears, blood plasma, blood serum, sputum, spinal fluid, serous fluid from a pleural cavity, nipple aspirate, lymph, tracheolar fluid, intestinal juice, genitourinary tract fluid, breast milk, semen, peritoneal fluid, cystic tumor fluid, amniotic fluid, or any combination thereof.

4. The method of any one of claims 1 to 3, wherein the sample is free of cells or cell debris.

5. The method of any one of claims 1 to 4, wherein the second component is fixed to a solid support.

6. The method of any one of claims 1 to 5, further comprising detecting the separated vesicle.

7. The method of any one of claims 1 to 6, further comprising contacting the separated vesicle with a ligand that has a binding affinity to the vesicle and detecting the ligand bound to the vesicle to analyze the vesicle.

8. The method of any one of claims 1 to 7, further comprising lysing the separated vesicle to separate a nucleic acid from the vesicle and amplifying the separated nucleic acid to analyze the nucleic acid of the vesicle.

9. The method of any one of claims 1 to 8, further comprising
contacting two or more ligands to the separated vesicle and
detecting the ligands bound to the vesicle to screen the ligands for binding affinity to the vesicle.

10. A composition or a kit for isolating a vesicle comprising:
a first component linked to a lipophilic molecule that can intervene into a lipid bilayer; and
a second component that binds to the first component.

11. The method of any one of claims 1 to 9, and the composition and kit of claim 10, wherein the lipophilic molecule comprises a fatty acid, sterol, glyceride or phospholipid.

12. The method, composition and kit of claim 11, wherein the phospholipid is 1,2-dihexadecanoyl-sn-glycero-3-phosphoethanolamine (DHPE), 1,2-dipalmitoyl-sn-glycero-3-phosphoethanolamine-N-(2,4-dinitrophenyl), or 1,2-dipalmitoyl-sn-glycero-3-phosphoethanolamine-N-[6-[(2,4-dinitrophenyl)amino]hexanoyl].

13. The method of any one of claims 1 to 9, 11 and 12 as well as the composition and kit of any one of claims 10 to 12, wherein the first component is an antibody, an antigen, a hapten, an enzyme, an enzyme substrate, an enzyme inhibitor, biotin, or avidin.

14. The method of any one of claims 1 to 9 and 11 to 13 as well as the composition and kit of any one of claims 10 to 13, wherein the second component is an antigen for an antibody, an antibody for an antigen, a hapten-binding protein for a hapten, an enzyme substrate or an enzyme inhibitor for an enzyme, an enzyme for an enzyme substrate or an enzyme inhibitor, avidin for biotin.

15. The method of any one of claims 1 to 9 and 11 to 14, as well as the composition and kit of any one of claims 10 to 14, wherein the vesicle is a liposome or a microvesicle.
